# EUROPÄISCHE PATENTSCHRIFT

(11) **EP 4 003 253 B1**
(45) Veröffentlichungstag und Bekanntmachung des Hinweises auf die Patenterteilung: **12.02.2025**
(21) Anmeldenummer: 20740573.9
(22) Anmeldetag: 10.07.2020
(51) Int. Cl.: A61F 13/01, B32B 5/24, A61F 13/00, D04H 1/485, D04H 1/498, D04H 3/105, B32B 5/06, B32B 5/18, B32B 5/08

(54) **WUNDREINIGUNGSARTIKEL**
WOUND CLEANING PRODUCT
PRODUIT DE NETTOYAGE DE PLAIE

(30) Priorität: 31.07.2019 DE 102019120712
(43) Veröffentlichungstag der Anmeldung: 01.06.2022
(73) Patentinhaber: Carl Freudenberg KG, 69469 Weinheim (DE)
(72) Erfinder: PEHR, Marc, 69514 Laudenbach (DE); LANG, Birthe, 69469 Weinheim (DE); SCHLESSELMANN, Bernd, 69469 Weinheim (DE)
(86) Internationale Anmeldenummer: PCT/EP2020/069478
(87) Internationale Veröffentlichungsnummer: WO 2021/018544

(56) Entgegenhaltungen:
- EP-A2- 0 211 131
- WO-A1-2013/113906
- WO-A1-2019/057256
- DE-U1- 202015 103 600
- US-A1- 2006 135 026

## Beschreibung

Die Erfindung betrifft einen Wundreinigungsartikel, umfassend ein Verbundmaterial, das eine Schaumlage und eine auf und in der Schaumlage angeordnete Faserlage aufweist sowie ein Verfahren zu seiner Herstellung.

In der Wundbehandlung spielt die Wundreinigung (Debridement), das heißt die Reinigung der Wunde von nekrotischem (abgestorbenen), infiziertem oder geschädigtem Gewebe, Fibrinbelägen, Wundexsudat und sonstigen Verschmutzungen eine integrale Rolle. Nekrotisches Gewebe und/oder eine starke Präsenz von Bakterien, können den Heilungsprozess einer Wunde stark behindern. Daher ist es medizinisch angeraten ein Debridement, insbesondere bei chronischen Wunden, zwischen jedem Verbandswechsel durchzuführen. Es gibt verschiedene Methoden, die zur Wundreinigung eingesetzt werden. Bei allen Methoden ist es essentiell, dass bei der Entfernung des abgestorbenen Gewebes bereits neu entstandenes Granulations- und junges Epithelgewebe, welche sich bei Wundschluss bilden, nicht beschädigt werden.

Eine Methode, die zunehmend verwendet wird, ist die mechanische Wundreinigung. Hierbei werden verschiedene Flächengebilde, zum Beispiel Textilien oder Schwämme, zur Reinigung der Wunde eingesetzt. Ein Vorteil der mechanischen Wundreinigung ist, dass sie einfach durchzuführen ist und auch in der Heimpflege von Pflegekräften angewendet werden kann.

Die Anforderungen an Flächengebilde für die mechanische Wundreinigung sind vielfältig. Zum einen müssen sie einen auf die Anwendung abgestimmten abrasiven Charakter haben, der ein sanftes schmerzfreies Entfernen nekrotischen Gewebes und von Belägen ermöglicht, zum anderen ein gutes Absorptionsvermögen aufweisen, das eine schnelle Aufnahme der gelösten Wundbelege ermöglicht und so eine erneute Kontamination der Wunde verhindert. Zusätzlich sollte sich das Flächengebilde auch zur Reinigung der Umgebungshaut eignen, wofür eine weniger abrasive Oberfläche geeignet ist. Des Weiteren sollte die Struktur flexibel sein, so dass ein Reinigen verschiedenster Wundgeometrien möglich ist, d.h. sowohl großflächiger Wunden als auch von Tunnelgängen.

Die Verwendung verschiedener Flächengebilde zur mechanischen Wundreinigung ist bereits bekannt.

Die WO 2019/028057 A1 beschreibt einen hydrophilen Schaum mit einem Feuchtegehalt zwischen 20 Gew.-% und 60 Gew.-% mit einer Struktur, die ein Debridement der Wunde erlaubt. Nachteilig hierbei ist, dass die Abrasivität über den Feuchtegehalt bestimmt wird. **In** der Anwendung ist dies nicht praktikabel, da es für das Pflegepersonal sehr aufwändig ist, die Wundspüllösung dosiert einzusetzen. Weiterhin nachteilig ist, dass keine Oberflächen mit unterschiedlichen Abrasivitäten zur Reinigung verfügbar sind.

**In** der WO 2019/057256 A1 sind zwei miteinander laminierte Schaumlagen dargelegt. Auf der einen Seite ist eine Schaumseite mit Vertiefungen und Einschnitten vorhanden. Es werden verschiedene Strukturen und Oberflächen zur Reinigung bereitgestellt. Nachteilig an diesem System ist, dass die Laminierung der beiden Schäume zu einer erhöhten Steifigkeit führen kann. Des Weiteren birgt ein unterschiedliches Quellverhalten der Schäume die Gefahr einer Delaminierung der Schichten.

Ein kommerziell erhältlicher Schaum zum mechanischen Debridement ist der Wundputzer^{®} von Ligasano^{®}. Der retikulierte hydrophobe Schaum zeigt eine gute Reinigungsleistung aufgrund seiner stark abrasiven Struktur. Diese Struktur kann aber auch zu starken Schmerzen beim Patienten während der Anwendung führen. Der retikulierte Schaum weist eine hohe Offenporigkeit auf, um feste und hoch viskose Wundbestandteile zu entfernen. Dies führt jedoch zu einem verringerten Absorptionsvermögen.

Neben Schäumen ist auch die Verwendung von textilen Flächengebilden zur Wundreinigung beschrieben.

Die EP 2 365 794 B1 beschreibt eine Wundreinigungseinrichtung, welche ein Tuch aufweist, oder ist, welche eine Trägerschicht mit auskragenden oder daran angeordneten Fäden aus synthetischen Fasern aufweist, die vorzugsweise abgeschnittene Enden bzw. Endflächen aufweisen. Nachteilig ist, dass die Reinigungswirkung durch die auskragenden Fasern erzielt wird. Dies führt schnell zu einem Verkleben der Faserenden, insbesondere bei Wunden mit viskosem Exsudat, was eine schnelle Erschöpfung der Kapazität des Tuches zur Folge hat. Dazu kommt, dass die zwischen den Fasern bereitgestellten Hohlräume, aufgrund ihrer länglichen Geometrie nicht so gut zur Aufnahme und Einschluss der Wundbestandteile geeignet sind.

Ein kommerziell verfügbares Produkt des vorgenannten Aufbaus, ist das Wundreinigungs-Produkt Debrisoft^{®}. Zusätzlich zu den obengenannten Nachteilen ist nachteilig, dass das Produkt harte versäuberte Kanten aufweist, die beim Kontakt mit dem Wundgrund starke Schmerzen verursachen können.

Die Kanten sind jedoch notwendig zum Zusammenhalten der textilen Struktur, da beim Zuschneiden des Produktes Fasern freigesetzt werden, die zu einer Kontamination der Wunde führen können. Dazu kommt, dass es eine hohe Steifigkeit hat, so dass es sich nur für den Einsatz auf flächigen Wunden eignet. Ferner wird Exsudat nur an der Oberfläche gebunden und nicht in der Struktur des Tuches aufgenommen. Schließlich ist es nur einseitig benutzbar.

Die EP 2777662 B1 beschreibt einen Velourvliesstoff für den Einsatz in der Wundreinigung, gekennzeichnet dadurch, dass die Fasern in Schlingen vorliegen. Hieran ist vorteilhaft, dass durch die Schlaufenstruktur Nekrosen und Wundabsonderungen gut entfernt und in der Struktur aufgenommen werden können. Nachteilig an der beschriebenen Erfindung ist, dass Abrasion und Absorption von der gleichen Lage durchgeführt werden und sich hierdurch nicht beliebig unabhängig voneinander variieren lassen.

US 20030079324 A1 beschreibt ein Verfahren zur Herstellung eines mittels Wasserstrahl verfestigten strukturierten durchlässigen Vliesstoffs mit hohem Absorptionsvermögen und guter Abrasion insbesondere im feuchten Zustand. Nachteilig ist hier die durch den Herstellungsprozess bedingte geringe Dicke des Materials, wodurch nur wenig Raum zur Aufnahme und zum Einschluss der Wundbestandteile zur Verfügung steht.

In der US 20170304485 A1 wird eine partikelhaltige poröse Vliesstoffmatrix, bestehend aus Polyolefinen sowie Glasfasern beschrieben, die eine Vielzahl an Mikroorganismen bindender Partikel enthält. Nachteilig an der Verwendung von Glasfasern ist, dass diese eine sehr hohe Abrasivität aufweisen, was sehr unangenehm für den Patienten sein kann. Des Weiteren stehen keine verschiedenen Oberflächen mit unterschiedlichen Flächeneigenschaften zur Verfügung.

Ein kommerziell verfügbares und weit verbreitetes mechanisches Wundreingungs-Produkt auf Faserbasis ist Baumwollgaze. Diese ist ein offenes Gewebe aus Baumwollgarnen und zeichnet sich zum einen durch ihren günstigen Preis und zum anderen durch eine hohe Versatilität aus. Nachteilig an diesem Produkt ist, dass es aufgrund der groben Garnstruktur in der Anwendung schmerzhaft für den Patienten sein kann. Darüber hinaus besteht die Gefahr einer Verunreinigung der Wunde durch sich aus dem Garn lösende Faserbestandteile, insbesondere beim Zuschneiden der Gaze.

Die WO 2013/113906 A1 beschreibt einen Wundpflegeartikel, aufweisend mindestens eine Oberfläche mit abrasiven Eigenschaften, die so ausgebildet ist, dass der Wundpflegeartikel geeignet ist, bei Relativbewegung desselben zu einer Wunde in der Wunde angeordnete Biofilme aufzubrechen und/oder die Wundexsudation anzuregen. Die Oberfläche mit abrasiven Eigenschaften kann ein Schaumstoff oder Fasern sein. Ebenfalls beschrieben ist ein Produkt, das sowohl einen Polyurethanschaum als auch auf diesen kaschiertes textiles Material enthält. Nachteilung an diesem Verbundmaterial ist seine hohe Steifigkeit und Delaminierungsneigung. Darüber hinaus wird ein Transport von Wundexsudat durch die Struktur durch die Kaschierung erschwert.

Ein kommerziell erhältliches Produkt des vorgenannten Aufbaus ist das Wundreinigungs-Produkt Cutimed^{®} DebriClean. Zusätzlich zu den obengenannten Nachteilen weist das Produkt den Nachteil auf, dass es harte Kanten sowie eine hohe Steifigkeit aufweist, wodurch es sich nur für großflächige Wunden eignet. Ferner ist es nur einseitig benutzbar.

Der Erfindung liegt die Aufgabe zu Grunde einen Wundreinigungsartikel bereitzustellen, mit dem die vorgenannten Nachteile zumindest teilweise ausgeräumt werden können. Insbesondere soll der Wundreinigungsartikel eine gute und schonende Wundreinigung ermöglichen. Dabei sollen nekrotisches Gewebe und Beläge ohne starke Krafteinwirkung entfernt werden können, so dass die Gefahr junges Granulationsgewebe zu verletzten minimiert wird. Darüber hinaus soll es mindestens zwei Bereiche unterschiedlicher Abrasivität aufweisen, so dass diese unabhängig voneinander für die Reinigung der Wunde als auch der Umgebungshaut optimiert werden können. Hierdurch kann mit einem Artikel sowohl die Wunde als auch die Umgebungshaut optimal versorgt werden. Außerdem soll der Artikel für verschiedene Wundgeometrien, beispielsweise großflächige oder tunnelförmige Wunden, einsetzbar sein.

Ferner soll der Artikel zuschneid- und einsetzbar sein, ohne signifikanten Verlust von Fasern.

DE202015103600U1 offenbart eine weitere Reinigungstextilie.

Diese Aufgabe wird gelöst durch einen Wundreinigungsartikel gemäß Anspruch 1, umfassend ein Verbundmaterial, das eine Schaumlage und eine auf und in der Schaumlage angeordnete Faserlage aufweist, wobei die Faserlage zumindest teilweise thermisch verfestigt ist und wobei Schaumlage und Faserlage durch Vernadeln miteinander verbunden sind, wobei Fasern der Faserlage in die Schaumlage eingedrungen sind und Kapillarkanäle ausbilden, die sich von der Faserlage in die Schaumlage erstrecken.

Der erfindungsgemäße Wundreinigungsartikel zeichnet sich dadurch aus, dass er eine Schaumlage und eine Faserlage aufweist, die durch Vernadeln miteinander verbunden sind. Durch diese mechanische Verbindung dringen Fasern der Faserlage in die Schaumlage ein und bilden Kapillarkanäle aus. Unter Kapillarkanälen werden erfindungsgemäß die zwischen den durch das Vernadeln ausgerichteten Fasern entstehenden langgestreckten Hohlräume verstanden. Die Kapillarkanäle erstrecken sich von der Faserlage in die Schaumlage und ermöglichen so einen guten und schnellen lagenübergreifenden Transport von Exsudat sowie dessen Einschluss im gesamten Wundreinigungsartikel.

Durch die Vernadelung von Faserlage und Schaumlage wird eine Struktur mit hoher Zugfestigkeit erzeugt. So kann das Verbundmaterial eine Höchstzugkraft/ Dehnung gemessen nach EN 29073-03 (nass) von mehr als 40 %, beispielsweise von 40 % bis 180 %, vorzugsweise mehr als 50 %, beispielsweise von 50 % bis 180 %, noch bevorzugter mehr als 60 %, beispielsweise von 60 % bis 180 %, noch bevorzugter mehr 70 %, beispielsweise von 70 % bis 180 %, noch bevorzugter mehr als 80 %, beispielsweise von 80 % bis 180 %, noch bevorzugter mehr als 90 %, beispielsweise von 90 % bis 180 %, und insbesondere mehr als 100 %, beispielsweise von 100 % bis 180 % aufweisen. Ferner kann ein zweiseitiges Material erhalten werden, bei dem auch beim Quellen und der damit verbundenen Ausdehnung der Lagen keine Gefahr der Delaminierung besteht. Ferner wurde überraschenderweise gefunden, dass durch die Vernadelung von Faserlage und Schaumlage ein Flüssigkeitstransport zwischen den Schichten ermöglicht wird, der einen schnellen Abtransport von Wundexsudat und ein sicheres Einschließen im Inneren der Struktur ermöglicht. Erfindungsgemäß durchdringen die Fasern der Faserlage die Schaumlage und ragen zumindest teilweise aus der der Faserlage abgewandten Seite der Schaumlage hervor. Dies ist vorteilhaft, da hierdurch eine besonders angenehme Haptik der Schaumseite erzielt werden kann. Darüber hinaus können die herausragenden Faserenden durch ihre hohe spezifische Oberfläche die Reinigungswirkung der Schaumlage noch weiter erhöhen.

Für manche Anwendungen ist die Verwendung der Faserlage als Reinigungsseite verglichen mit der Schaumlage vorteilhaft, da Fasern in der Regel weniger abrasiv als Schäume sind, so dass eine sanfte Reinigung der Umgebungshaut möglich ist. Darüber hinaus bieten die Fasern eine höhere spezifische Oberfläche als der Schaum zur Reinigung und Adsorption kleiner gelöster Wundbestandteile und Bakterien.

Ferner wurde gefunden, dass der erfindungsgemäße Wundreinigungsartikel eine sanfte Wundreinigung ermöglicht. Insbesondere gelingt die Ablösung viskoser und verkrusteter Bestandteile bereits bei geringem Druck. Des Weiteren ermöglicht der erfindungsgemäße Wundreinigungsartikel verschiedene Oberflächenstrukturen in einem einzigen Material, so dass verschiedene Reinigungsbedürfnisse mit einem einzigen Produkt adressiert werden können.

Erfindungsgemäß ist die Faserlage thermisch verfestigt. Durch die thermische Verfestigung können die Fasern gut fixiert werden, so dass die Gefahr eines Faserverlustes und somit Kontamination der Wunde reduziert werden kann. Darüber hinaus kann über den Grad der thermischen Verfestigung die Abrasivität gezielt eingestellt werden.

Die thermische Verfestigung erfolgt vorzugsweise dadurch, dass die Faserlage vor dem Aufbringen auf die Schaumlage, thermisch verfestigt wird. Hierdurch kann eine Beeinträchtigung der Schaumlage durch das Erhitzen verhindert werden.

In praktischen Versuchen hat sich gezeitgt, dass es besonders zweckmäßig ist, wenn die Faserlage eine Faserlage ist, die ohne Komprimierung thermisch verfestigt wurde. Dies hat den Vorteil, dass das Porenvolumen auch nach dem Verfestigen zu hohen Anteilen zur Aufnahme von Wundbestandteilen zur Verfügung steht. Des Weiteren kann hierdurch eine Faserlage mit einer rauhen Oberfläche erhalten werden, was das Lösen von schwer löslichen Belägen in der Wunde ermöglicht. Darüberhinaus erlaubt dies den Erhalt der Flexibilität der Faserlage. Ein thermisches Verfestigen ohne Komprimierung kann beispielsweise dadurch erfolgen, dadurch dass die Faserlage mit Heißluft behandelt wird, beispielsweise im Durchluftofen.

Weiter bevorzugt ist, wenn die Faserlage eine flächig thermisch verfestigte Faserlage ist. Dies ist gegenüber nicht flächig thermisch verfestigten Faserlagen, wie beispielsweise Punkt-Kalandrierten Faserlagen von Vorteil, da so verhindert werden kann, dass sich einzelne Fasern aus der Faserlage lösen und die Wunde kontaminieren.

In einer bevorzugten Ausführungsform der Erfindung ist die Faserlage ein Vliesstoff, insbesondere ein Vliesstoff nach DIN EN ISO 9092:2019-08, ein Gewebe, ein Gewirke und/oder ein Gestrick, wobei die vorgenannten Faserlagen jeweilst zumindest teilweise thermisch verfestigt sind. Besonders bevorzugt ist die Faserlage ein zumindest teilweise thermisch verfestigter Vliesstoff, da dieser aufgrund seiner offenen Struktur ein besonders gutes Eindringen der Fasern in die Schaumlage erlaubt.

Die Faserlage kann die verschiedensten Fasern aufweisen. Bevorzugt sind synthetische Fasern, da diese sich gut sterilisieren lassen. Weiter bevorzugt sind hydrophobe Fasern, d.h. Fasern, die zumindest an ihrer Oberfläche aus Polymeren gefertigt sind, deren Oberflächenenergie niedriger als 50 mJ/m² beträgt. Besonders geeignete hydrophobe Fasern sind Polyester- und/oder Polyolefinfasern. An hydrophoben Fasern ist vorteilhaft, dass sie das Anbinden und somit Entfernen von Bakterien ermöglichen. Die Anbindung von Bakterien an hydrophobe Fasern ist beispielsweise beschrieben in N. Edwards et al. Role of surface energy and nanoroughness in the removal efficiency of bacterial contamination by nonwoven wipes from frequently touched surfaces, Science and Technology of advanced materials, 2017, Vol. 18, No. 1 197 -209. Zur Ausbildung der Gerüststruktur weist die Faserlage vorzugsweise Matrixfasern auf. Unter Matrixfasern sind erfindungsgemäß Fasern zu verstehen, die nicht, oder nur zu einem geringen Teil thermisch verschmolzen vorliegen. Bevorzugt sind thermoplastische Matrixfasern, die einen Schmelzpunkt der am niedrigsten schmelzenden Faserkomponente von über 170 °C, beispielsweise von 170 °C bis 250 °C, noch bevorzugter von über 200 °C aufweisen. In einer Ausführungsform bestehen die Matrixfasern aus einer Faserkomponente. In einer weiteren bevorzugten Ausführungsform bestehen die Matrixfasern aus mehreren Faserkomponenten. Besonders bevorzugt sind Vollprofilfasern, Multilobalvollprofilfasern, Hohlfasern und/oder Vollprofilbikomponentenfasern (z.B. Kern/Mantel, Side-by-Side). Sofern die Faserlage Bindefasern aufweist, ist es vorteilhaft, wenn der Schmelzpunkt der in der Matrixfaser enthaltenen Faserkomponente mit dem niedrigsten Schmelzpunkt über dem Schmelzpunkt der in der Bindefaser enthaltenen Faserkomponente mit dem höchsten Schmelzpunkt liegt. Vorzugsweise liegt der Schmelzpunkt der Faserkomponente mit dem niedrigsten Schmelzpunkt in der Matrixfaser mindestens 10 °C, besonders bevorzugt mindestens 30 °C, über dem Schmelzpunkt der Faserkomponente mit dem höchsten Schmelzpunkt in der Bindefaser. Geeignete Polymerklassen zur Herstellung der Matrixfasern können unter anderen Polyester, Polyamide, Polyolefine, Polyacrylnitril, Cellulose und/oder Polyvinylalkohole sein.

Besonders geeignete Matrixasern sind hydrophobe Matrixfasern.

Der Anteil der Matrixfasern in der Faserlage beträgt vorzugsweise mindestens 20 Gew.-%, beispielsweise von 20 Gew.-% bis 90 Gew.-% und/oder von 20 Gew.-% bis 80 Gew.-%, und/oder von 20 Gew.-% bis 70 Gew.-%, noch bevorzugter mindestens 25 Gew.-%, beispielsweise von 25 Gew.-% bis 80 Gew.-% und/oder von 25 Gew.-% bis 70 Gew.-%, noch bevorzugter mindestens 30 Gew.-%, beispielsweise von 30 Gew.-% bis 70 Gew.-% auf, jeweils bezogen auf das Gesamtgewicht der Faserlage.

In einer bevorzugten Ausführungsform weist die Faserlage ein Bindemittel, beispielsweise thermoplastische Binder und/oder Bindefasern auf. Bindefasern sind Fasern, die zumindest teilweise verschmolzen sind und hierdurch Bindepunkte zwischen den Fasern schaffen. Hierdurch ist eine thermische Verfestigung und eine gezielte Einstellung der Abrasivität der Faserlage möglich. Vorzugsweise weist mindestens eine schmelzbare Faserkomponente der Bindefaser, insbesondere eine außen angeordnete schmelzbare Faserkomponente, einen Schmelzpunkt auf, der niedriger liegt als der Schmelzpunkt anderer in der Faserlage enthaltener Faserkomponenten, insbesondere niedriger als der Schmelzpunkt der am niedrigsten schmelzenden Faserkomponente der Matrixfasern.

Weist die Bindefaser mehrere schmelzbare Faserkomponenten auf, so liegt der Schmelzpunkt der am höchsten schmelzenden Faserkomponente der Bindefaser bevorzugt mehr als 10 °C, besonders bevorzugt mehr als 30 °C unter dem Schmelzpunkt der am niedrigsten schmelzenden Faserkomponente der Matrixfasern. Geeignete Bindefasern weisen einen Schmelzpunkt der am höchsten schmelzenden Faserkomponente von unter 250 °C, beispielsweise von 100 °C bis 200 °C, noch bevorzugter von unter 180 °C, beispielsweise von 100 °C bis 180 °C, insbesondere unter 175 °C, beispielsweise von 100 °C bis 175 °C auf.

Bevorzugte schmelzbare Komponenten in Bindefasern sind Polyolefin, Polyester, Polyamid und/oder Gemische hiervon sowie Copolymere wie Ethylen-Vinylacetat-Copolymere. Ebenfalls bevorzugte Bindefasern sind Bikomponentenfasern, insbesondere Bikomponentenfasern, die Polyolefin, Polyester, Ethylen-Vinylacetat-Copolymere, Polybutylenterephtalat, und/oder Gemische hiervon als außen angeordnete Faserkomponente enthalten. Die Bindefasern können unterschiedliche Querschnittsgeometrien wie Vollprofilfaser-, Multilobalvollprofilfaser-, Hohlfaser-, Vollprofilbikomponentenfaser- (z.B. Kern/Mantel, Side-by-Side) geometrien aufweisen. Bevorzugt sind Schmelzbindefasern in Form von Vollprofilfasern.

Besonders geeignete Bindemittel sind hydrophobe Bindefasern, insbesondere Polyester- und/oder Polyolefinfasern.

Der Anteil der Bindefasern in der Faserlage beträgt vorzugsweise mindestens 10 Gew.-%, beispielsweise von 10 Gew.-% bis 80 Gew.-% und/oder von 10 Gew.-% bis 70 Gew.-%, und/oder von 10 Gew.-% bis 60 Gew.-%, noch bevorzugter mindestens 25 Gew.-%, beispielsweise von 25 Gew.-% bis 80 Gew.-% und/oder von 25 Gew.-% bis 70 Gew.-%, und/oder von 25 Gew.-% bis 60 Gew.-%, noch bevorzugter mindestens 30 Gew.-%, beispielsweise von 30 Gew.-% bis 80 Gew.-% und/oder von 30 Gew.-% bis 70 Gew.-%, und/oder von 30 Gew.-% bis 60 Gew.-%, jeweils bezogen auf das Gesamtgewicht der Faserlage.

Thermoplastische Binder können Co-Polyamid, Co-Polyester, Polyolefine, Polyvinylalkohol (PVA), Ethylen-Vinylacetat (EVA), thermoplastisches Polyurethan (TPU), Polycaprolacton, Terpolymere und/oder Mischungen hiervon sein. Bevorzugt enthält der thermoplastische Binder die vorgenannten Polymere in einer Menge von mehr als 50 Gew.-%, besonders bevorzugt von mehr als 70 Gew.-%, insbesondere mehr als 90 Gew.-%, jeweils bezogen auf das Gesamtgewicht des Binders. Bevorzugte thermoplastische Binder weisen einen Schmelzpunkt von 90 °C bis 200 °C auf. Besonders geeignete thermoplastische Binder sind hydrophob, d.h. aus Polymeren gefertigt, deren Oberflächenenergie niedriger als 50 mJ/m2 beträgt.

In einer bevorzugten Ausführungsform sind die in der Faserlage enthaltenen Fasern, insbesondere die Matrixfasern und/oder die Bindefasern, Stapelfasern, bevorzugt mit einer Stapellänge zwischen 20 mm und 150 mm, noch bevorzugter zwischen 30 mm und 90 mm und insbesondere zwischen 40 mm und 70 mm.

Der Fasertiter der in der Faserlage enthaltenen Fasern, insbesondere der Matrixfasern und/oder Bindefasern liegt vorzugsweise im Bereich von 0,9 dtex bis 100 dtex (g/10.000 m). Noch bevorzugter liegt der Fasertiter zwischen 1,5 dtex und 30 dtex, insbesondere zwischen 3 dtex und 11 dtex.

Bevorzugt weist die Faserlage eine mittlere Dicke, mikroskopisch bestimmt, von mindestens 1,5 mm, beispielsweise von 1,5 mm bis 10 mm, noch bevorzugter von mindestens 2 mm, beispielsweise von 2 mm bis 10 mm, und/oder von 4 mm bis 10 mm und/oder von 2 mm bis 5 mm und/oder von 5 mm bis 8 mm auf.

Das Flächengewicht der Faserlage beträgt vorzugsweise 50 g/m² bis 400 g/m², noch bevorzugter 50 g/m² bis 350 g/m², noch bevorzugter 50 g/m² bis 300 g/m², noch bevorzugter 50 g/m² bis 250 g/m², insbesondere 50 g/m² bis 200 g/m².

Als Schaumlage können erfindungsgemäß die verschiedensten Schäume, insbesondere Polymerschäume eingesetzt werden. Vorzugsweise basiert die Schaum lage auf Polyurethanschaum, beispielsweise Polyetherpolyurethan- oder Polyesterpolyurethanschaum, Polyetheresterpolyurethanschaum, Polyvinylacetatschaum, Polyvinylalkoholschaum oder auf Mischungen dieser Schäume. Der Begriff "basierend auf" meint dabei mehr als 50 Gew.-%, noch bevorzugter mehr als 70 Gew.-%, insbesondere mehr als 90 Gew.-%, bezogen auf den Polymeranteil der Schaumlage.

Besonders bevorzugt enthält die Schaumlage einen hydrophilen Polymerschaum, das heißt einen Schaum mit einer Absorptionskapazität von mindestens 4 g/g, beispielsweise von 4 g/g bis 50 g/g, vorzugsweise von 4 g/g bis 30 g/g und noch bevorzugter von 4 g/g bis 25 g/g in einem Anteil von mehr als 50 Gew.-%, noch bevorzugter mehr als 70 Gew.-%, insbesondere mehr als 90 Gew.-%, bezogen auf den Polymeranteil der Schaumlage. Ebenfalls bevorzugt ist ein Polymerschaum, der aus hydrophilen Polymeren, bevorzugt hydrophilen Polyurethanen, insbesondere hydrophilen Polyurethanen wie in der WO2018/007093 beschrieben, gefertigt ist. Ein hydrophiler Polymerschaum erlaubt eine schnelle Aufnahme von viskosen Wundbestandteilen. Ferner ermöglicht er ein Einweichen der Nekrosen, wenn er zuvor mit Wundspüllösung getränkt und auf der Wunde appliziert wird. Ganz besonders bevorzugt ist ein Polyurethanschaum, da dieser eine hohe Hydrophilie mit einer guten Elastizität und Retention verbindet. Vorteilhaft an einer guten Retention ist ein sicherer Einschluss entfernter Wundbestandteile, so dass eine erneute Kontaminierung der Wunde beziehungsweise der Wundumgebung verhindert werden kann.

In einer besonders bevorzugten Ausführungsform der Erfindung ist die Schaumlage offenzellig. Unter dem Begriff "offenzellig" ist erfindungsgemäß zu verstehen, dass die Mehrzahl der Poren durch Porenöffnungen miteinander verbunden ist. Vorteilhaft hieran ist, dass aufgenommene Wundbestandteile gut in der Struktur aufgenommen und homogen verteilt werden können.

In einer weiteren bevorzugten Ausführungsform weist die Schaumlage mindestens eine offenzellige Oberfläche auf. Unter dem Begriff "offenzellige Oberfläche" ist erfindungsgemäß zu verstehen, dass der prozentuale Anteil der Fläche von geöffneten Poren zur Gesamtoberfläche der Schaumlage mindestens 50 %, beispielsweise 50 % bis 98 %, noch bevorzugter mindestens 60 %, beispielsweise 60 % bis 98 %, insbesondere mindestens 70 %, beispielsweise 70 % bis 98 % beträgt. Vorteilhaft an einer derartigen offenzelligen Schaumlage ist, dass sie einen leicht abrasiven Charakter hat, der ein sanftes Lösen von Belägen ermöglicht und zugleich gelöste Wundbestandteile gut in der Struktur zurückhalten kann, so dass eine Kontamination der Wunde verhindert wird.

In einer weiteren Ausführungsform weist die Schaumlage einen retikulierten Schaum auf. Bei retikulierten Schäumen wird die Offenzelligkeit durch das Aufreißen der Porenwände mittels einer Gasexplosion in den Schaumporen erzeugt.

Das Flächengewicht der Schaumlage beträgt vorzugsweise 50 g/m² bis 600 g/m², noch bevorzugter 80 g/m² bis 500 g/m², noch bevorzugter 100 g/m² bis 400 g/m², noch bevorzugter 120 g/m² bis 350 g/m², insbesondere 120 g/m² bis 250 g/m².

Die mittlere Dicke der Schaumlage beträgt bevorzugt 1 mm bis 15 mm, besonders bevorzugt 2 mm bis 10 mm und besonders bevorzugt von 3 mm bis 8 mm.

Die mittlere Porengröße der Schaumlage beträgt mindestens 0,2 mm, beispielsweise 0,2 mm bis 3 mm, noch bevorzugter mindestens 0,25 mm, beispielsweise 0,25 mm bis 2,5 mm, insbesondere mindestens 0,3 mm, beispielsweise 0,3 mm bis 2,2 mm.

In einer weiteren bevorzugten Ausführungsform der Erfindung beträgt die mittlere Dichte der Schaumlage mindestens 50 kg/m³, vorzugsweise von 70 kg/m³ bis 150 kg/m³ und insbesondere von 90 kg/m³ bis 150 kg/m³.

In einer bevorzugten Ausführungsform weist das Verbundmaterial eine Absorptionskapazität von mindestens 4 g/g, beispielsweise von 4 g/g bis 50 g/g, vorzugsweise von 4 g/g bis 30 g/g und noch bevorzugter von 6 g/g bis 25 g/g auf.

Das Flächengewicht des Verbundmaterials beträgt vorzugsweise 100 g/m² bis 1000 g/m², noch bevorzugter 200 g/m² bis 900 g/m², noch bevorzugter 200 g/m² bis 600 g/m², noch bevorzugter 200 g/m² bis 500 g/m², insbesondere 250 g/m² bis 450 g/m².

In einer bevorzugten Ausführungsform der Erfindung weist das Verbundmaterial eine hydrophile Schaumlage auf in Kombination mit einer hydrophoben Faserlage. Unter einer hydrophilen Schaumlage wird eine Schaumlage mit einer Absorptionskapazität von mindestens 4 g/g, beispielsweise von 4 g/g bis 50 g/g, vorzugsweise von 4 g/g bis 30 g/g und noch bevorzugter von 4 g/g bis 25 g/g verstanden. Ebenfalls bevorzugt ist eine Schaumlage, die aus einem Polymerschaum besteht, der hydrophile Polymere, bevorzugt hydrophile Polyurethane, insbesondere hydrophile Polyurethane wie in der WO2018/007093 beschrieben, enthält, vorzugsweise in einem Anteil von mindestens 50 Gew.-%, noch bevorzugter mindestens 70 Gew.-%, insbesondere mindestens 90 Gew.-% bezogen auf das Gesamtgewicht der Schaum lage.

In einer besonders bevorzugten Ausführungsform weist das Verbundmaterial eine hydrophile Schaumlage auf in Kombination mit einer hydrophoben Faserlage, wobei hydrophobe Fasern der Faserlage die hydrophile Schaumlage durchdringen und zumindest teilweise aus der der Faserlage abgewandten Seite der Schaumlage hervorragen. An dieser Ausführungsform ist vorteilhaft, dass eine bifunktionelle Oberfläche auf der Schaumlage zur Verfügung gestellt wird, die durch den hydrophilen Charakter der Schaumlage eine schnelle Aufnahme von viskosen Wundbestandteilen ermöglicht und gleichzeitig durch die vergleichsweise hohe spezifische Oberfläche der herausragenden Fasern die Adsorption von Bakterien und anderen hydrophoben Bestandteilen ermöglicht.

Unter einer hydrophoben Faserlage wird eine Faserlage verstanden, die hydrophobe Fasern, d.h. Fasern, die aus Polymeren gefertigt sind, deren Oberflächenenergie niedriger als 50 mJ/m² beträgt, enthält, vorzugsweise in einem Anteil von mindestens 50 Gew.-%, noch bevorzugter mindestens 70 Gew.-%, insbesondere mindestens 90 Gew.-%, bezogen auf das Gesamtgewicht der Faserlage. Besonders geeignete hydrophobe Fasern sind Polyester- und/oder Polyolefinfasern.

Für die Anwendung zur Wundreinigung ist der Wundreinigungsartikel vorzugsweise steril.

Ein weiterer Gegenstand der vorliegenden Erfindung betrifft die Herstellung eines erfindungsgemäßen Wundreinigungsartikels umfassend folgende Schritte:
A Anordnen einer zumindest teilweise thermisch verfestigten Faserlage auf einer Schaumlage;
B Vernadeln von Schaumlage und Faserlage derart, dass sich ein Verbundmaterial ausbildet, in dem Fasern der Faserlage in die Schaumlage eingedrungen sind und Kapillarkanäle ausbilden, die sich von der Faserlage in die Schaumlage erstrecken.

Ein weiterer Gegenstand der vorliegenden Erfindung betrifft eine alternative Herstellung eines erfindungsgemäßen Wundreinigungsartikels umfassend folgende Schritte:
A Anordnen einer Faserlage auf einer Schaumlage;
B Vernadeln von Schaumlage und Faserlage derart, dass sich ein Verbundmaterial ausbildet, in dem Fasern der Faserlage in die Schaumlage eingedrungen sind und Kapillarkanäle ausbilden, die sich von der Faserlage in die Schaumlage erstrecken.
C Thermisches Verfestigen des Verbundmaterials.

### Messmethoden:

Bestimmung offenzellige Oberfläche: Die Offenzelligkeit der Oberfläche wird durch die Aufnahme eines lichtmikroskopischen Bildes (Interferenzmikroskop Lichtquelle grün) der Oberfläche der Schaumlage auf einer Fläche von 1 cm x 1 cm und durch anschließende optische Auswertung bestimmt. Bestimmt wird der prozentuale Anteil der Fläche von geöffneten Poren zur Gesamtoberfläche der Schaum lage.

Porendurchmesser der Poren in der Schaumlage: Der Porendurchmesser wird durch optische Auswertung von Lichtmikroskopie-Aufnahmen durch Anlegen eines Außenkreises bestimmt. Der Porendurchmesser entspricht dem Durchmesser des Außenkreises. Es wird über die Auswertung von mindestens 10 Poren gemittelt.

Absorptionskapazität: Für Absorptionsmessungen wird eine Testlösung, wie in BS EN 13726-1:2002 beschrieben, verwendet. Eine 100 cm² große Probe wird zuerst gewogen (W1), danach in die Testlösung gelegt und dort für mindestens zehn Minuten belassen. Anschließend wird die Probe vorsichtig an einer Ecke gefasst, ohne die Probe zu quetschen, und für zwei Minuten abtropfen gelassen. Danach wird erneut das Gewicht bestimmt (W2). Die Absorptionskapazität berechnet sich nun durch das Teilen der Betragsdifferenz von W2 und W1 durch das anfängliche Gewicht W1 als Absorption [g] pro [g]. Dichte der Schaumlage: Die Dichte wird ermittelt, indem eine Probe ausgeschnitten, gewogen und die Dicke ermittelt wird. Anschließend wird das Volumen durch Multiplikation von Dicke mit Fläche der Probe berechnet und schließlich das Gewicht durch das Volumen geteilt.

Dicke der Schaumlage: Die Dicke der Schaumlage wird mikroskopisch vor dem Vernadeln mit der Faserlage bestimmt.

### Figurenbeschreibung

### Kurzbeschreibung der Zeichnung

In den Zeichnungen zeigen:
Fig. 1 den Durchschnitt einer CT-Aufnahme eines erfindungsgemäßen Verbundmaterials,
Fig. 2 den Querschnitt einer Lichtmikroskopie-Aufnahme eines erfindungsgemäßen Verbundmaterials nach einer Wundreinigung,
Fig. 3 den Querschnitt einer Rasterelektronenmikroskopie-Aufnahme einer Baumwollgaze nach einer Wundreinigung,
Fig. 4 den Querschnitt einer Rasterelektronenmikroskopie-Aufnahme eines erfindungsgemäßen Wundreinigungsmaterials nach einer Wundreinigung,
Fig. 5 die definierte Bewegung eines Wischzyklus zur Reinigung einer Testwunde auf Schweinehaut,
Fig. 6 den Querschnitt einer Rasterelektronenmikroskopie-Aufnahme des Produktes Debrisoft ^{®} nach einer Wundreinigung.

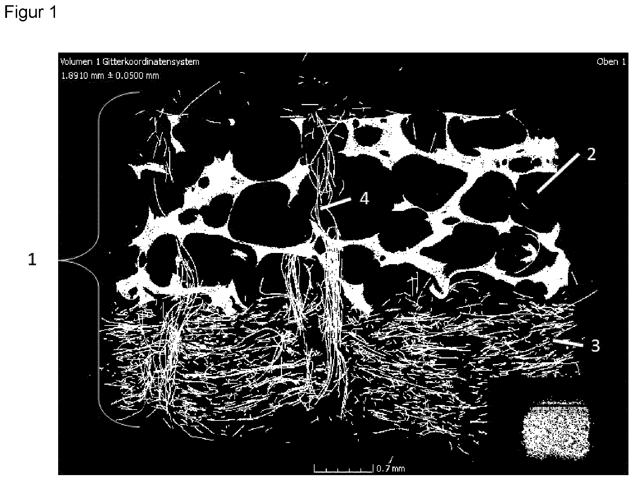

Fig. 1 zeigt den Durchschnitt einer CT Aufnahme eines erfindungsgemäßen Verbundmaterials 1, das eine Schaumlage 2 und eine auf dieser angeordnete Faserlage 3 aufweist. Schaumlage 2 und Faserlage 3 sind durch Vernadeln miteinander verbunden, so dass Fasern der Faserlage 3 in die Schaumlage 2 eingedrungen sind und in dieser Kapillarkanäle 4 ausbilden. Man erkennt, dass die Kapillarkanäle 4 sich von der Faserlage 3 in die Schaumlage 2 erstrecken.

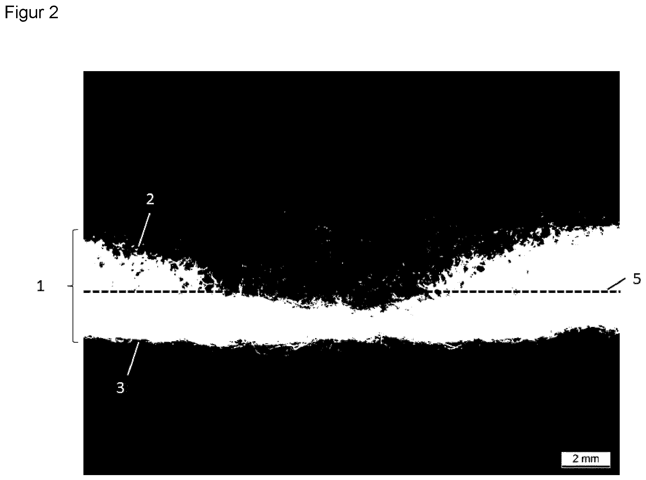

Fig. 2 zeigt den Querschnitt einer Lichtmikroskopie Aufnahme eines erfindungsgemäßen Verbundmaterials 1 nach einer Wundreinigung mit der Schaumseite. In der Figur ist die Grenzfläche von Schaumlage 2 und Faserlage 3 als Grenzlinie 5 illustriert. Man erkennt deutlich, wie Wundbestandteile tief in der Schaumlage 2 absorbiert werden und die Grenzlinie 5 überschreiten, das heißt bis in die Faserlage eingedrungen 3 sind.

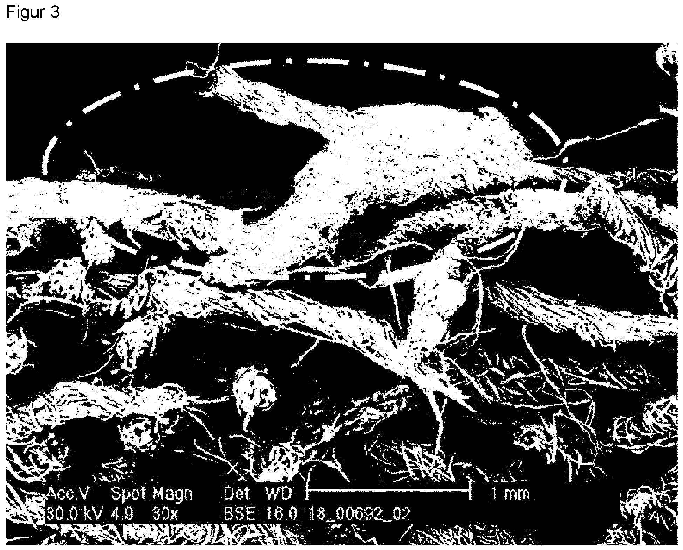

Fig. 3 zeigt den Querschnitt einer Rasterelektronenmikroskopie Aufnahme einer Baumwollgaze nach einer Wundreinigung. Man erkennt deutlich, wie die Wundbestandteile lediglich auf der Oberfläche der Baumwollgaze festgehalten werden (Bereich innerhalb Punkt-Strich Linie).

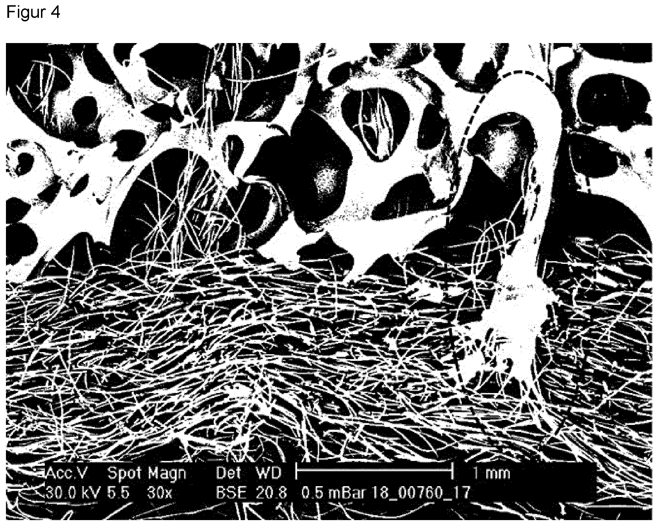

Fig. 4 zeigt den Querschnitt einer Rasterelektronenmikroskopie Aufnahme eines erfindungsgemäßen Wundreinigungsmaterials nach einer Wundreinigung. Es ist deutlich zu erkennen, wie Wundexsudat entlang der durch Fasern gebildeten Kapillarkanäle von der Schaumlage in die Faserlage geleitet wird (Markiert innerhalb gepunkteter Linie).

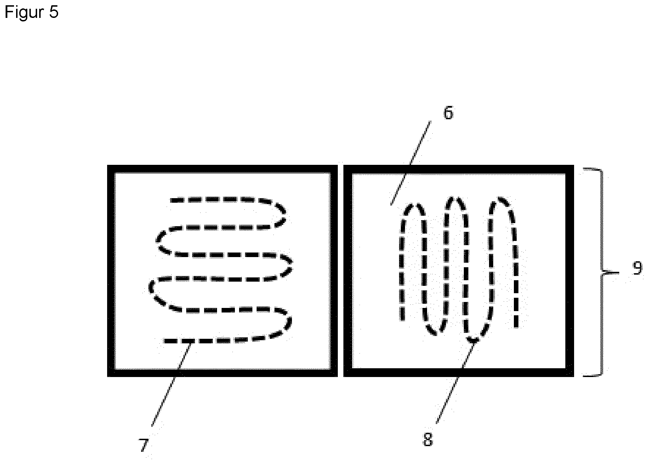

Fig. 5 zeigt die definierte Bewegung eines Wischzyklus zur Reinigung einer Testwunde auf Schweinehaut. Die Reinigung der Wundfläche 6 erfolgt mit geringem Druck (zur Simulation einer schonenden Reinigung) und in einer definierten horizontalen 7 und vertikalen 8 Bewegung, die einen Wischzyklus 9 ergeben.

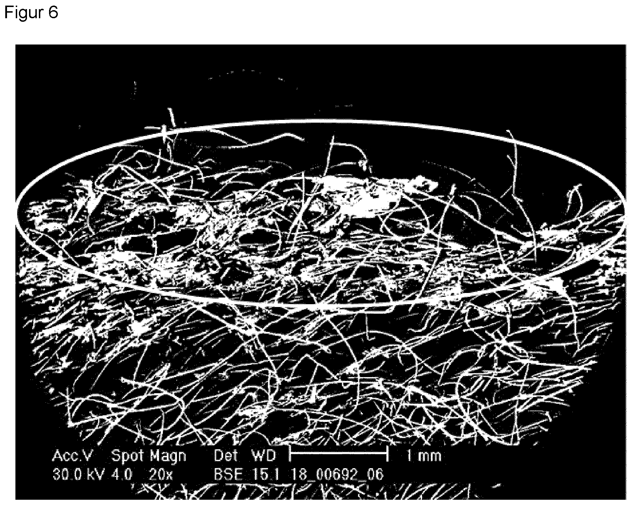

Fig. 6 zeigt den Querschnitt einer Rasterelektronenmikroskopie Aufnahme des Produktes Debrisoft ^{®} nach einer Wundreinigung. Man erkennt deutlich, wie die Wundbestandteile lediglich auf der Oberfläche festgehalten werden (Bereich innerhalb Ovals).

### Beispiele

### Beispiel 1: Herstellen einer Schaumlage

Eine Wasserphase wird für die Schaumherstellung durch Lösen/Dispergieren des Tensids Pluronic F87 in einer Konzentration von 0,5 Gew.-% hergestellt. Gleichzeitig wird eine Teflonform mit einer ausreichenden Tiefe, um einen Schaum von 7 mm Dicke z erzeugen, mit Gießpapier ausgekleidet. Das Präpolymer Hypol 2001 wird in einer Konzentration von 40 Gew.-% zu der Wasserphase gegeben und bei Raumtemperatur mit einer Dispergierscheibe (1600 U/min) gemischt. Das erhaltene Gemisch wird sofort in die Gießform gegossen. Es wird gegen Luft geschäumt und für 10 Minuten ausgehärtet. Danach wird das Gießpapier entfernt und bei einer Temperatur von 150 °C für 3 Stunden getrocknet.

### Beispiel 2: Herstellen einer thermisch verfestigten Faserlage

Zur Herstellung der thermisch verfestigen Faserlage werden Stapelfasern verwendet: 70 Gew.-% Polyester Fasern (Grisuten^{®} der Märkischen Faser GmbH) mit einem Fasertiter von 1,7 dtex und einer Faserlänge von 60 mm und 30 Gew.-% einer Polyethythlenterephtalat/ Polyethylen Kern-Mantel Bindefaser (Trevira^{®} 256 der Trevira GmbH) mit einem Fasertiter von 3 dtex und einer Faserlänge von 50 mm. Diese Stapelfasern werden mittels einer Krempel nach dem Fachmann bekannten Verfahren in eine Faserlage gelegt. Das Flächengewicht der Faserlage beträgt 150 g/m². Anschließend wird die Faserlage in einem Heißluftofen bei 150 °C und einer Geschwindigkeit von 2 m/min thermisch abgebunden.

### Beispiel 3: Herstellen eines Wundreinigungsartikels durch mechanisches Vernadeln von Faserlage und Schaumlage

Zur Herstellung des erfindungsgemäßen Wundreinigungsartikels werden eine offenporige Schaumlage aus Beispiel 1 und eine thermisch verfestigte Faserlage aus Beispiel 2 bereitgestellt. In einem Nadelprozess wird die Vlieslage mit einer Einstichdichte von 100/ cm² und Einstichtiefe von 10 mm gegen die Schaumlage vernadelt. Dabei befindet sich die Oberfläche der Vlieslage, die im Ofen von der Bandseite abgewendet war, auf der der Schaum lagenoberfläche abgewandten Seite. Durch den Vernadelungsprozess werden Schaumlage und Faserlage miteinander verbunden. Auf diese Weise werden sowohl ein mechanisch stabiler Verbund, als auch eine leicht faserige Oberfläche auf dem Schaum erzeugt. Darüber hinaus durchdringen die Fasern der Faserlage die Schaumlage, bilden in der Schaumlage Kapillarkanäle aus und ragen zumindest teilweise aus der der Faserlage abgewandten Seite der Schaumlage hervor. Das erhaltene Verbundmaterial weist auf der Schaumlage eine höhere Abrasivität als auf der Faserlage auf. Durch die aus der Schaumlage herausragenden Fasern, weist die Schaumlage eine angenehmere Haptik als die reine Schaumlage auf. Zur Herstellung eines Wundreinigungsartikels wird das Verbundmaterial konfektioniert. Dabei tritt kein signifikanter Verlust von Fasern auf.

### Beispiel 4: Bestimmung der mechanischen Stabilität des Verbundmaterials aus Beispiel 3

Die mechanische Stabilität wird als Zugfestigkeit des erfindungsgemäßen Verbundmaterials mit Hilfe einer Zugprüfung nach EN 29073-03 (Abweichungen: Keine Konditionierung und Abzugsgeschwindigkeit 200 mm/min) in Längsrichtung mit einer Probenkörpergröße von 300 x 50 mm getestet. Es wurde die Trocken- und Nassreißfestigkeit bestimmt, da Wundreinigungsmaterialien in der Regel feucht angewendet werden. Zur Beurteilung der Nassreißfestigkeit wurden die Probekörper für circa 30 Minuten in Leitungswasser gelegt und anschließend leicht ausgedrückt und hängend für 5 - 10 Minuten abtropfen gelassen.

Tabelle 1 zeigt im Vergleich die Höchstzugkraft und Höchstzugkraft Dehnung für einen offenzelligen Schaum, das erfindungsgemäße Verbundmaterial und die Standard Baumwollgaze. Das erfindungsgemäße Verbundmaterial weist eine deutlich höhere Höchstzugkraft im nassen und im trocknen Zustand im Vergleich zu einem reinen offenzelligen Schaum auf. Durch die Durchdringung der Schaumlage durch die Faserlage wird die Schaumlage mechanisch verstärkt und die Zugfestigkeit erhöht. Im trockenen Zustand wird dadurch die Festigkeit einer Standard Baumwollkompresse erzielt. Im nassen Zustand weist das erfindungsgemäße Verbundmaterial eine geringere jedoch ausreichende Höchstzugkraft als die Standard Baumwollgaze auf. Die Höchstzugkraft Dehnung liegt hingegen deutlich über der Baumwollgaze. Hierdurch eignet sich das erfindungsgemäße Verbundmaterial hervorragend zur feuchten Wundreinigung, da die hohe Dehnbarkeit ein Reißen während der Reinigung verhindern kann. Ferner bietet die erhöhte Dehnung den Vorteil, dass sich das Verbundmaterial besser an den Wundgrund anpassen kann.

**Tabelle 1: Vergleich mittlere Höchstzugkraft und Höchstzugkraft Dehnung für offenzelligen Schaum, ein erfindungsgemäßes Verbundmaterial und Standard Baumwollgaze**

| | Troc cken | | Nass | |
|---|---|---|---|---|
| | Höchstzugkraft [N] | Höchstzugkraft Dehnung [%] | Höchstzugkraft [N] | Höchstzugkraft Dehnung [%] |
| Offenzellige Schaumlage [2,5 mm] | 22,1 | 165,8 | 10,2 | 58,9 |
| Erfindungsgemäßes Verbundmaterial [~4 mm] | 87,4 | 135,3 | 76,0 | 130,3 |
| Standard Baumwollgaze [< 1 mm] | 83,3 | 5,0 | 143,7 | 8,2 |

### Beispiel 5: Bestimmung der Reinigungsleistung des Wundreinigungsartikels in einem ex-vivo Wundmodell

Das in Beispiel 3 hergestellte Verbundmaterial wurde durch Stanzen auf eine Größe von 10 cm x 10 cm zu einem erfindungsgemäßen Wundreinigungsartikel konfektioniert. Die Reinigungsleistung des erfindungsgemäßen Wundreinigungsartikels wurde mit Hilfe eines ex-vivo Wundmodels auf Schweinehaut bewertet. Die zu reinigende Wunde wurde wie folgt vorbereitet: Zunächst wurde die Schweinehaut mit der Hautinnenseite nach oben in einem Rahmen (Fläche für Wunde 10 cm x 10 cm) fixiert, mit einem Skalpell definiert in Form eines Gittermusters eingeritzt. Anschließend wurde die Haut mit einem Flammbierbrenner leicht verbrannt (20 Sekunden, mittlere Flamme, Abstand ca. 20 cm), so dass durch Aufklaffen der Schnitte ein unebener Wundgrund entsteht. Austretendes Fett wurde vorsichtig mit einem Papiertuch durch Tupfen entfernt. Anschließend wurden 4 mL Eiweißlösung (100 g/L Eiweißpulver (bspw. Body&Fit Egg White Powder in Phosphat gepufferter Salzlösung (8,0 g/L Natriumchlorid (NaCl), 0,2 g/L Kaliumchlorid (KCI), 1,42 g/L Dinatriumhydrogenphosphat (Na2HPO4) oder 1,78 g/L Dinatriumhydrogenphosphat Dihydrat (Na2HPO4• 2 H2O), 0,27 g/L Kaliumdihydrogenphosphat (KH2PO4)) gelöst) aufgetragen und erneut mit Hilfe eines Flammbierbrenners eingebrannt (30 Sekunden, mittlere Flamme, Abstand ca. 20 cm). Die Eiweißlösung eignet sich zur Simulierung von Fibrinbelägen in der Wunde. In einem letzten Schritt werden 2 g künstliche Exsudatlösung (5 % Blanose, 40 g/L Eiweißpulver, 1 g/L Zucker, Kunstblut (150 Tropfen auf 300 mL, 2g/L Sonnenblumen Öl, 3,7 g/L Natrium Carbonat, 3 g/L Quarzsand alles gelöst in PBS) mittels eines Holzspachtels auf die Wunde aufgetragen und erneut eingebrannt, bis sich (teilweise) eine schwarze Kruste bildet (30 Sekunden, mittlere Flamme, Abstand ca. 20 cm). Die simulierte Wunde wird 2 Stunden bei Raumtemperatur ruhen gelassen, damit zuvor appliziertes Exsudat nicht leicht wieder entfernt werden kann.

Zur Reinigung wurde der Wundreinigungsartikel auf der Schaumseite (10 cm x 10 cm) mit 5 Sprühstößen mit Wasser angefeuchtet und mit der Schaumseite zur Wundoberfläche zeigend, eine Minute auf die Wunde gelegt. Anschließend erfolgte die Reinigung der Wundfläche mit geringem Druck (zur Simulation einer schonenden Reinigung) und in einer definierten horizontalen und vertikalen Bewegung, die einen Wischzyklus ergeben (siehe Figur 5). Die Reinigung erfolgt für 3 Minuten mit 11 Wischzyklen.

Die Auswertung der Reinigungsleistung erfolgte über Bildanalyse der verbrannten (schwarzen) Bereiche vor und nach der Wundreinigung. Die präparierten Schweinehäute wurden mit einer Spiegelreflexkamera (Canon D70) mit Festbrennweitenobjektiv (60 mm) mit Hilfe eines Reprostativs aufgenommen. Dabei wurde darauf geachtet, dass die Beleuchtung und der Abstand (resultierende Vergrößerung) zwischen Objekt und Objektiv über alle Aufnahmen gleich waren. Im Zuge der Untersuchungen wurde ein Metallrahmen zur Glättung der Schweinehäute aufgelegt. Über eine Schwellwertsetzung und einen 15 x 15 Pixel Medianfilter wurden die schwarzen Bereiche im Bild ausfindig gemacht und grün markiert. Für alle Bilder wurde eine Obergrenze von 80 im Histogramm gewählt. Dies, und das gleichbleibende Aufnahmesetup stellen sicher, dass die Ergebnisse aller Proben vergleichbar sind. Als letzter Schritt wurde der sinnvoll auszuwertende Bereich des Bildes begrenzt (ROI). Aufgrund der Kalibrierung der Bilder bei der Aufnahme war es anschließend möglich die markierten Bereiche als Flächenanteile in µm anzugeben.

**Tabelle 2: Vergleich der Reinigungsleistung von Baumwollgaze, Debrisoft^{®} und eines erfindungsgemäßen Wundreinigungsartikels**

| | Fläche vor Reinigung [mm²] | Fläche nach Reinigung [mm²] | Reinigungsleistung in [%] |
|---|---|---|---|
| Baumwollgaze | 629,0 | 286,0 | 54,5 |
| Baumwollgaze | 1491,0 | 1010,0 | 32,3 |
| Debrisoft^{®} | 3162,0 | 1276,0 | 59,6 |
| Debrisoft^{®} Erfindungsgemäßer | 3374,0 | 1289,0 | 61,7 |
| Wundreinigungsartikel Erfindungsgemäßer | 2924,0 | 922,0 | 68,5 |
| Wundreinigungsartikel | 59,0 | 14,0 | 76,3 |

### Beispiel 6 Bestimmung der Aufnahme von Wundbestandteilen durch den erfindungsgemäßen Wundreinigungsartikel aus Beispiel 5

Zur Bestimmung der Aufnahme von Wundbestandteilen im Wundreinigungsartikel wurden Licht- und Rasterelektronen-Mikroskopieaufnahmen angefertigt. Man erkennt deutlich (Figur 2), dass ein Transport von Wundbestandteilen über die Grenze der Schaumlage hinaus in die Faserlage erfolgt. Dahingegen erfolgt die Absorption bei Baumwollgaze (Figur 3 Bereich innerhalb Strich-Punkt Oval) und Debrisoft (Figur 6 Bereich innerhalb Oval) nur auf der Oberfläche, bzw. im oberen Teil der Struktur. Ferner ist ersichtlich (Figur 3 gestrichelter Bereich), dass die Kapillarkanäle einen lagenübergreifen Transport sowie Einschluss von Exsudat im gesamten Wundreinigungsartikel ermöglichen.

Der erfindungsgemäße Wundreinigungsartikel wird im Hinblick auf seine Absorptionsfähigkeit im Vergleich zu Baumwollgaze und dem Produkt Debrisoft^{®} nach BS EN 13726-1:2002 getestet. Es wurde gefunden, dass er eine erhöhte Absorption der wässrigen Testlösung im Vergleich zu Standard Baumwollgaze und zu dem Produkt Debrisoft^{®} zeigt (vgl. Tabelle 3). Hieran ist vorteilhaft, dass mehr Wundbestandteile sowie Flüssigkeiten wie z.B. zur Unterstützung der Wundreinigung eingesetzte Spüllösungen und/oder Wundexsudat innerhalb einer Wundreinigungsanwendung aufgenommen werden können.

**Tabelle 3: Vergleich der Absorption von Baumwollgaze, Debrisoft^{®} und einem erfindungsgemäßen Wundreinigungsartikel**

| | Absorption [g/g] |
|---|---|
| Baumwollgaze | 5,2 |
| Debrisoft ^{®} | 5,9 |
| Erfindungsgemäßer Wundreinigungsartikel | 6,5 |

## Patentansprüche

1. Wundreinigungsartikel, umfassend ein Verbundmaterial (1), das eine Schaumlage (2) und eine auf und in der Schaumlage (2) angeordnete Faserlage (3) aufweist, wobei die Faserlage (3) zumindest teilweise thermisch verfestigt ist und die Schaumlage (2) und Faserlage (3) durch Vernadeln miteinander verbunden sind, wobei Fasern der Faserlage (3) in die Schaumlage (2) eingedrungen sind und Kapillarkanäle (4) ausbilden, die sich von der Faserlage (3) in die Schaumlage (2) erstrecken **dadurch gekennzeichnet, dass** die Fasern der Faserlage (3) die Schaumlage (2) durchdringen und zumindest teilweise aus der der Faserlage (3) abgewandten Seite der Schaumlage (2) hervorragen.

2. Wundreinigungsartikel nach Anspruch 1, **dadurch gekennzeichnet, dass** die Faserlage (3) synthetische Fasern aufweist.

3. Wundreinigungsartikel nach Anspruch 1 oder 2, **dadurch gekennzeichnet, dass** die Faserlage (3) Matrixfasern und ein Bindemittel, insbesondere Bindefasern aufweist.

4. Wundreinigungsartikel nach einem oder mehreren der vorangehenden Ansprüche, **dadurch gekennzeichnet, dass** die Faserlage (3) hydrophobe Fasern, insbesondere Polyester und/oder Polyolefinfasern aufweist.

5. Wundreinigungsartikel nach einem oder mehreren der vorangehenden Ansprüche, **dadurch gekennzeichnet, dass** die in der Faserlage (3) enthaltenen Fasern, insbesondere die Matrixfasern und/oder die Bindefasern, Stapelfasern sind, bevorzugt mit einer Stapellänge zwischen 20 mm und 150 mm.

6. Wundreinigungsartikel nach einem oder mehreren der vorangehenden Ansprüche, **dadurch gekennzeichnet, dass** die Schaumlage (2) einen hydrophilen Polymerschaum, in einem Anteil von mehr als 50 Gew.-%, noch bevorzugter mehr als 70 Gew.-%, insbesondere mehr als 90 Gew.% bezogen auf den Polymeranteil der Schaumlage (2) aufweist.

7. Wundreinigungsartikel nach einem oder mehreren der vorangehenden Ansprüche, **dadurch gekennzeichnet, dass** die Schaumlage (2) offenzellig ist.

8. Wundreinigungsartikel nach einem oder mehreren der vorangehenden Ansprüche, **dadurch gekennzeichnet, dass** die Schaumlage (2) mindestens eine offenzellige Oberfläche aufweist.

9. Wundreinigungsartikel nach einem oder mehreren der vorangehenden Ansprüche, **gekennzeichnet durch** eine Höchstzugkraft/ Dehnung gemessen nach EN 29073-03 (nass) von mehr als 40 %.

10. Wundreinigungsartikel nach einem oder mehreren der vorangehenden Ansprüche, **dadurch gekennzeichnet, dass** das Verbundmaterial (1) eine hydrophile Schaumlage (2) in Kombination mit einer hydrophoben Faserlage (3) aufweist.

11. Wundreinigungsartikel nach Anspruch 10, **dadurch gekennzeichnet, dass** hydrophobe Fasern der Faserlage (3) die hydrophile Schaumlage (2) durchdringen und zumindest teilweise aus der der Faserlage (3) abgewandten Seite der Schaumlage (2) hervorragen.

12. Wundreinigungsartikel nach einem oder mehreren der vorangehenden Ansprüche, **dadurch gekennzeichnet, dass** er steril ist.

13. Verfahren zur Herstellung eines erfindungsgemäßen Wundreinigungsartikels umfassend folgende Schritte:
A Anordnen einer zumindest teilweise thermisch verfestigten Faserlage (3) auf einer Schaumlage (2);
B Vernadeln von Schaumlage (2) und Faserlage (3) derart, dass sich ein Verbundmaterial (1) ausbildet, in dem Fasern der Faserlage (3) in die Schaumlage (2) eingedrungen sind und Kapillarkanäle (4) ausbilden, die sich von der Faserlage (3) in die Schaumlage (2) erstrecken.

14. Verfahren zur Herstellung eines erfindungsgemäßen Wundreinigungsartikels umfassend folgende Schritte:
A Anordnen einer Faserlage (3) auf einer Schaumlage (2);
B Vernadeln von Schaumlage (2) und Faserlage (3) derart, dass sich ein Verbundmaterial (1) ausbildet, in dem Fasern der Faserlage (3) in die Schaumlage (2) eingedrungen sind und Kapillarkanäle (4) ausbilden, die sich von der Faserlage (3) in die Schaumlage (2) erstrecken.
C Thermisches Verfestigen des Verbundmaterials (1).

## Claims

1. Wound cleaning article comprising a composite material (1) which has a foam layer (2) and a fibre layer (3) arranged on and in the foam layer (2), wherein the fibre layer (3) is at least partially thermally consolidated and the foam layer (2) and fibre layer (3) are connected to one another by needling, where fibres of the fibre layer (3) have penetrated into the foam layer (2) and form capillary channels (4) which extend from the fibre layer (3) into the foam layer (2), **characterized in that** the fibres of the fibre layer (3) penetrate the foam layer (2) and protrude at least partially from that side of the foam layer (2) that faces away from the fibre layer (3).

2. Wound cleaning article according to Claim 1, **characterized in that** the fibre layer (3) comprises synthetic fibres.

3. Wound cleaning article according to Claim 1 or 2, **characterized in that** the fibre layer (3) comprises matrix fibres and a binder, more particularly binding fibres.

4. Wound cleaning article according to one or more of the preceding claims, **characterized in that** the fibre layer (3) comprises hydrophobic fibres, more particularly polyesters and/or polyolefin fibres.

5. Wound cleaning article according to one or more of the preceding claims, **characterized in that** the fibres contained in the fibre layer (3), more particularly the matrix fibres and/or the binding fibres, are staple fibres, preferably having a staple length of between 20 mm and 150 mm.

6. Wound cleaning article according to one or more of the preceding claims, **characterized in that** the foam layer (2) comprises a hydrophilic polymer foam in a fraction of more than 50% by weight, more preferably more than 70% by weight, more particularly more than 90% by weight, based on the polymer fraction of the foam layer (2).

7. Wound cleaning article according to one or more of the preceding claims, **characterized in that** the foam layer (2) is open-celled.

8. Wound cleaning article according to one or more of the preceding claims, **characterized in that** the foam layer (2) has at least one open-celled surface.

9. Wound cleaning article according to one or more of the preceding claims, **characterized by** a tensile strength/elongation, measured according to EN 29073-03 (wet), of more than 40%.

10. Wound cleaning article according to one or more of the preceding claims, **characterized in that** the composite material (1) comprises a hydrophilic foam layer (2) in combination with a hydrophobic fibre layer (3).

11. Wound cleaning article according to Claim 10, **characterized in that** hydrophobic fibres of the fibre layer (3) penetrate the hydrophilic foam layer (2) and protrude at least partially from that side of the foam layer (2) that faces away from the fibre layer (3).

12. Wound cleaning article according to one or more of the preceding claims, **characterized in that** it is sterile.

13. Method for producing a wound cleaning article of the invention, comprising steps as follows:
A arranging an at least partially thermally consolidated fibre layer (3) on a foam layer (2);
B needling foam layer (2) and fibre layer (3) in such a way as to form a composite material (1) in which fibres of the fibre layer (3) have penetrated into the foam layer (2) and form capillary channels (4) which extend from the fibre layer (3) into the foam layer (2).

14. Method for producing a wound cleaning article of the invention, comprising steps as follows:
A arranging a fibre layer (3) on a foam layer (2);
B needling foam layer (2) and fibre layer (3) in such a way as to form a composite material (1) in which fibres of the fibre layer (3) have penetrated into the foam layer (2) and form capillary channels (4) which extend from the fibre layer (3) into the foam layer (2);
C thermally consolidating the composite material (1) .

## Revendications

1. Article de nettoyage de plaies, comprenant un matériau composite (1) qui présente une couche de mousse (2) et une couche de fibres (3) agencée sur et dans la couche de mousse (2), la couche de fibres (3) étant au moins partiellement consolidée thermiquement et la couche de mousse (2) et la couche de fibres (3) étant reliées entre elles par aiguilletage, des fibres de la couche de fibres (3) ayant pénétré dans la couche de mousse (2) et formant des canaux capillaires (4) qui s'étendent de la couche de fibres (3) dans la couche de mousse (2), **caractérisé en ce que** les fibres de la couche de fibres (3) traversent la couche de mousse (2) et dépassent au moins partiellement du côté de la couche de mousse (2) détourné de la couche de fibres (3).

2. Article de nettoyage de plaies selon la revendication 1, **caractérisé en ce que** la couche de fibres (3) présente des fibres synthétiques.

3. Article de nettoyage de plaies selon la revendication 1 ou 2, **caractérisé en ce que** la couche de fibres (3) présente des fibres de matrice et un liant, notamment des fibres de liaison.

4. Article de nettoyage de plaies selon une ou plusieurs des revendications précédentes, **caractérisé en ce que** la couche de fibres (3) présente des fibres hydrophobes, notamment des fibres de polyester et/ou de polyoléfine.

5. Article de nettoyage de plaies selon une ou plusieurs des revendications précédentes, **caractérisé en ce que** les fibres contenues dans la couche de fibres (3), notamment les fibres de matrice et/ou les fibres de liaison, sont des fibres discontinues, de préférence avec une longueur comprise entre 20 mm et 150 mm.

6. Article de nettoyage de plaies selon une ou plusieurs des revendications précédentes, **caractérisé en ce que** la couche de mousse (2) présente une mousse polymère hydrophile, en une proportion de plus de 50 % en poids, de préférence encore de plus de 70 % en poids, notamment de plus de 90 % en poids par rapport à la proportion de polymère de la couche de mousse (2).

7. Article de nettoyage de plaies selon une ou plusieurs des revendications précédentes, **caractérisé en ce que** la couche de mousse (2) est à cellules ouvertes.

8. Article de nettoyage de plaies selon une ou plusieurs des revendications précédentes, **caractérisé en ce que** la couche de mousse (2) présente au moins une surface à cellules ouvertes.

9. Article de nettoyage de plaies selon une ou plusieurs des revendications précédentes, **caractérisé par** une force de traction maximale/un allongement mesuré(e) selon EN 29073-03 (humide) de plus de 40 %.

10. Article de nettoyage de plaies selon une ou plusieurs des revendications précédentes, **caractérisé en ce que** le matériau composite (1) présente une couche de mousse hydrophile (2) en combinaison avec une couche de fibres hydrophobe (3).

11. Article de nettoyage de plaies selon la revendication 10, **caractérisé en ce que** les fibres hydrophobes de la couche de fibres (3) pénètrent dans la couche de mousse hydrophile (2) et dépassent au moins partiellement du côté de la couche de mousse (2) détourné de la couche de fibres (3).

12. Article de nettoyage de plaies selon une ou plusieurs des revendications précédentes, **caractérisé en ce qu'**il est stérile.

13. Procédé de fabrication d'un article de nettoyage de plaies selon l'invention, comprenant les étapes suivantes :
A l'agencement d'une couche de fibres (3) au moins partiellement consolidée thermiquement sur une couche de mousse (2) ;
B l'aiguilletage de la couche de mousse (2) et de la couche de fibres (3) de telle sorte qu'un matériau composite (1) se forme, dans lequel des fibres de la couche de fibres (3) ont pénétré dans la couche de mousse (2) et forment des canaux capillaires (4) qui s'étendent de la couche de fibres (3) dans la couche de mousse (2).

14. Procédé de fabrication d'un article de nettoyage de plaie selon l'invention, comprenant les étapes suivantes :
A l'agencement d'une couche de fibres (3) sur une couche de mousse (2) ;
B l'aiguilletage de la couche de mousse (2) et de la couche de fibres (3) de telle sorte qu'un matériau composite (1) se forme, dans lequel des fibres de la couche de fibres (3) ont pénétré dans la couche de mousse (2) et forment des canaux capillaires (4) qui s'étendent de la couche de fibres (3) dans la couche de mousse (2) ;
C la consolidation thermique du matériau composite (1).
